# EUROPEAN PATENT APPLICATION

(11) **EP 4 653 032 A1**
(43) Date of publication of application: **26.11.2025**
(21) Application number: 23916725.7
(22) Date of filing: 18.01.2023
(51) Int. Cl.: A61M 5/32, A61M 5/31, A61M 5/178

(54) **NEEDLE OUTPUT BUFFER MECHANISM AND SYRINGE COMPRISING NEEDLE OUTPUT BUFFER MECHANISM**

(71) Applicant: C C Biotechnology Corporation, Tainan City, Taiwan 70955 (TW)
(72) Inventor: YEH, Chin-Min, Tainan City Taiwan (TW); WANG, Chih-Wei, Tainan City Taiwan (TW)
(74) Representative: Savi, Massimiliano
(86) International application number: PCT/CN2023/072872
(87) International publication number: WO 2024/152238

(57) **Abstract**

A needle output buffer mechanism (B) and a syringe comprising the needle output buffer mechanism (B). The syringe comprises the needle output buffer mechanism (B) and an injection propulsion mechanism (C), and is capable of mounting a barrel group (A). The needle output buffer mechanism (B) is connected to a barrel (10) of the barrel group (A) by means of a buffer member (20) with an elastic arm (202). The needle output buffer mechanism (B) can link with the barrel group (A), which is ejected by the injection propulsion mechanism (C), and produces elastic collision in the syringe by utilizing the elastic arm (202) of the buffer member (20) so as to absorb an impact force, thereby providing a buffer protection performance for the ejected barrel group (A) and ensuring that the barrel group (A) is prevented from being damaged due to an extremely large impact force during ejection.

## Description

### Field of the Invention

The present invention relates to a syringe, especially to a needle output buffer mechanism and a syringe comprising the needle output buffer mechanism.

### Description of the Prior Art(s)

In currently known syringes, to facilitate users to perform subcutaneous injection of liquid medicine, in addition to a hollow outer casing for mounting a barrel group, an injection propulsion device is mounted in the outer casing and is installed behind the barrel group, so as to use the injection propulsion device to allow the user to push the barrel group for injection.

In the overall structural design of the syringe as described above, the injection propulsion device can be used to drive the barrel group in a front section of the syringe to eject a needle to allow the needle of a barrel to pierce into the subcutaneous muscle of the human body to inject the liquid medicine. However, since the injection propulsion device has to provide enough ejection force to drive the barrel group to eject the needle to pierce into the subcutaneous muscle of the human body, the barrel may be damaged due to strong impact force between the barrel group ejecting the needle forward and components in the syringe during ejection. Especially when the barrel of the barrel group is made of glass, the glass barrel is connected with the injection propulsion device through a rear end portion with a wing. When the barrel group is ejected, there is a lack of impact protection mechanism in the syringe, such that the wing and other parts of the glass barrel break easily due to the strong impact force and connection structure between the injection propulsion device and the barrel is also damaged, which makes it difficult for the injection propulsion device to perform an injection action normally. Thus, the conventional structure of the syringe needs to be further improved.

### Content of the Invention

The technical problem to be solved by the present invention is that: by providing a needle output buffer mechanism and a syringe comprising the needle output buffer mechanism to solve the problem that when an injection propulsion mechanism of the conventional syringe ejects a barrel group to output a needle, a barrel in the barrel group is easily damaged due to strong impact force.

The technical solution proposed in the present invention is to provide a needle output buffer mechanism which is mounted in a syringe. The needle output buffer mechanism is connected with a barrel group. When the barrel group is ejected to output the needle by an injection propulsion mechanism in the syringe, the needle output buffer mechanism and the barrel group are movable together. The needle output buffer mechanism comprises:
a buffer member connected with the barrel group and the buffer member including at least one elastic arm; the buffer member providing buffering elasticity to the barrel group that is ejected to output the needle through elastic contact of the elastic arm in the syringe.

The beneficial effects that the needle output buffer mechanism of the present invention provides are: with the resilient arms of the buffer member being connected with the barrel of the barrel group and with the needle output buffer mechanism and the barrel group that is ejected to output the needle by the injection propulsion mechanism being movable together, the elastic arms of the buffer member resiliently collide in the syringe to absorb impact force, so as to provide a perfect buffer protection mechanism for the barrel group that is ejected to output the needle and to ensure that the barrel would not be broken due to strong impact when outputting the needle.

In the needle output buffer mechanism, the buffer member includes a member base and two elastic arms symmetrically mounted on a front end of the member base; the front end of the member base has two hooking portions arranged symmetrically, the two elastic arms are symmetrically arranged and disposed by two sides of the two hooking portions; the two hooking portions hook on two barrel wing portions on a rear end of a barrel of the barrel group; the two elastic arms are positioned outside the two barrel wing portions and protrude from front sides of the barrel wing portions. Thus, the elastic arms of the buffer member can provide balanced impact absorption performance during the ejection of the barrel group.

In the needle output buffer mechanism, each of the elastic arms includes a curved concave portion bending backward and two curved convex portions symmetrically disposed on two sides of the curved concave portion and bending forward; the elastic arm is connected with the member base through the two curved convex portions, so as to form a space between the elastic arm and the member base for buffering deformation.

In the needle output buffer mechanism, a spring plate may be further mounted on a rear side of the buffer member; the spring plate has a center hole and multiple resilient abutting portions disposed around the center hole; each of the resilient abutting portions is touchable by the injection propulsion mechanism at different injection positions to make sounds as sound prompt; and/or with each of the resilient abutting portions providing resistant force to the injection propulsion mechanism that is driven to move forward, an effect of slowing down infusion speed can be achieved.

Another technical solution proposed in the present invention is to provide a syringe with a barrel group containing liquid medicine mounted therein. The syringe comprises:
an outer casing; an interior thereof having a front accommodating room and a rear accommodating room arranged front to back and communicating with each other; the barrel group mounted in the outer casing and being movable in the front accommodating room and the rear accommodating room;
an injection propulsion mechanism mounted in the rear accommodating room of the outer casing and protruding out from a rear end of the outer casing; the injection propulsion mechanism disposed behind the barrel group and being operable to eject the barrel group forward from the front accommodating room of the outer casing to output a needle and inject the liquid medicine;
a needle output buffer mechanism mounted in the rear accommodating room of the outer casing; the needle output buffer mechanism connected to a rear end of a barrel of the barrel group and being movable along with the barrel group; the needle output buffer mechanism including a buffer member; the buffer member including at least one elastic arm; the buffer member providing buffering elasticity to the barrel group that is ejected to output the needle through elastic contact of the elastic arm in the syringe;
a needle retracting mechanism mounted in the rear accommodating room of the outer casing and positioned in front of the needle output buffer mechanism; the barrel group passing through the needle retracting mechanism; after the injection propulsion mechanism is operated to eject the barrel group and the needle output buffer mechanism connected thereto to complete a needle ejection action and an injection action of the liquid medicine, the needle retracting mechanism is triggered to drive the barrel group and the needle output buffer mechanism that is connected with the barrel group and the injection propulsion mechanism move backward until the needle of the barrel group returns to the outer casing.

The beneficial effects that the syringe of the present invention provides are: in addition to the effects included in the aforementioned needle output buffer mechanism, the outer casing can be used for mounting the needle output buffer mechanism, the injection propulsion mechanism and the needle retracting mechanism. Thus, the injection propulsion mechanism is able to eject the barrel group that is connected with the needle output buffer mechanism to output the needle and to perform an injection action of the liquid medicine. Meanwhile, the needle output buffer mechanism provides buffering protection to the barrel of the barrel group that is ejected. After the injection action of the liquid medicine is completed, the needle retracting mechanism automatically retracts the barrel group back into the outer casing, so as to achieve effects of retracting and protecting a needle tip.

In the syringe of the present invention, a safety lock mechanism that is connected with the injection propulsion mechanism may be further mounted in the outer casing. By limiting movements of the injection propulsion mechanism with the safety lock mechanism, when the safety lock mechanism is in a locked position, the injection propulsion mechanism is prevented from being accidentally triggered; when the safety lock mechanism is switched to an unlocked position, the injection propulsion mechanism is able to be operated to perform a needle output action and the injection action.

### Description of the Drawings

The following drawings are only intended to illustrate and explain the present invention and do not limit the scope of the present invention, wherein:
Fig. 1 is a perspective view of a combination of an embodiment of a needle output buffer mechanism of the present invention and a barrel group and some mechanisms in a syringe;
Fig. 2 is a perspective view viewed from another angle of Fig. 1;
Fig. 3 is a perspective view of the embodiment of the needle output buffer mechanism in Figs. 1 and 2;
Fig. 4 is a perspective view viewed from another angle of the embodiment of the needle output buffer mechanism in Figs. 1 and 2;
Fig. 5 is a perspective view of another embodiment of a needle output buffer mechanism of the present invention;
Fig. 6 is a perspective view viewing from another angle of said another embodiment of the needle output buffer mechanism in Fig. 5;
Fig. 7 is a perspective view of a combination of said another embodiment of the needle output buffer mechanism in Figs. 5 and 6 and a barrel group and some mechanisms in a syringe;
Fig. 8 is a perspective appearance view of an embodiment of a syringe of the present invention;
Fig. 9 is an exploded perspective view of the embodiment of the syringe of the present invention;
Fig. 10 is an exploded perspective view viewed from another angle of the embodiment of the syringe in Fig. 9;
Fig. 11 is a cross-sectional side view of the embodiment of the syringe in Figs. 9 and 10;
Fig. 12 is a cross-sectional top view of the embodiment of the syringe in Figs. 9 and 10;
Fig. 13 is a perspective view of the embodiment of the syringe in Figs. 9 and 10, wherein an outer casing and a safety lock mechanism are removed;
Fig. 14 is a partial perspective view of the embodiment of the syringe in Figs. 9 and 10, wherein the safety lock mechanism and an injection button are in a locked state;
Fig. 15 is a partial perspective view of the safety lock mechanism in Fig. 14, wherein a locking ring is rotated to an unlocked state;
Fig. 16 is an exploded perspective view of a needle output buffer mechanism, a needle retracting mechanism, an injection push rod and a barrel group of the syringe in Figs. 9 to 12;
Fig. 17 is a perspective view viewed from another angle of Fig. 16;
Fig. 18 is an exploded perspective view of the needle output buffer mechanism with a spring plate added therein, the needle retracting mechanism, the injection push rod and the barrel group of the syringe in Figs. 9 to 12;
Fig. 19 is a perspective view viewed from another angle of Fig. 18;
Fig. 20 is a reference view (1) of the embodiment of the syringe in Figs. 10 to 13 in state of use;
Fig. 21 is a reference view (2) of the embodiment of the syringe in Figs. 10 to 13 in state of use;
Fig. 22 is a reference view (3) of the embodiment of the syringe in Figs. 10 to 13 in state of use;
Fig. 23 is a reference view (4) of the embodiment of the syringe in Figs. 10 to 13 in state of use;
Fig. 24 is a reference view (5) of the embodiment of the syringe in Figs. 10 to 13 in state of use;
Fig. 25 is a reference view (6) of the embodiment of the syringe in Figs. 10 to 13 in state of use;
Fig. 26 is a cross-sectional side view of the embodiment of the syringe in Fig. 21, wherein a needle sheath is taken out of the outer casing by using a needle sheath pulling device;
Fig. 27 is a cross-sectional side view of the embodiment of the syringe in Fig. 22, wherein the locking ring of the safety lock mechanism is rotated to the unlocked state;
Fig. 28 is a cross-sectional view (1) of the embodiment of the syringe in Fig. 23, wherein the injection button is pressed to output a needle;
Fig. 29 is a cross-sectional view (2) of the embodiment of the syringe in Fig. 23, wherein the injection button is pressed to output a needle;
Fig. 30 is a cross-sectional view of the embodiment of the syringe in Fig. 24, wherein an injection propulsion mechanism drives a piston of the barrel group for injection and infusion;
Fig. 31 is a cross-sectional view of the embodiment of the syringe in Fig. 24, wherein the needle retracting mechanism drives the barrel group to perform a needle retracting action.

### Description of the Reference Numbers:

A: barrel group; 10: barrel; 101: barrel wing portion; 11: needle; 12: piston; 13: needle sheath; B: needle output buffer mechanism; 20: buffer member; 201: member base; 202: elastic arm; 203: hooking portion; 204: aligning guide slot; 21: spring plate; 211: resilient abutting portion; 212: notch; 30: outer casing; 301: front accommodating room; 302: rear accommodating room; 303: annular retaining wall; 304: screw groove; 305: positioning buckle potion; 31: outer protective layer; 311: injection direction indicating mark; 312: lock position mark; 313: unlock position mark; 32: needle sheath pulling device; 321: sleeving portion; 322: hooking portion; 323: operation end portion; C: injection propulsion mechanism; 40: inner sleeve; 401: inner sleeve base; 402: axial supporting portion; 403: stop arm; 41: injection button; 411: pressing end; 412: expanding arm; 413: guiding protrusion; 42: injection push rod; 421: rod part; 422:baffle part; 423: rear rod end; 424:pressing plate; 43: rear resilient element; D: needle retracting mechanism; 50: front fastener; 501: fastener base; 502: ratchet arm; 503: screw end; 5031: screw rib; 504: accommodating room; 505: end wall; 506: fastener bore; 51: front resilient element; E: safety lock mechanism; 60: inner liner tube; 601: rotating guide groove; 602: locking area; 603: unlocking area; 61: locking ring; 612: connecting end; 613: rotating guide protrusion; 614: indicating portion; 615: guiding groove.

### Description of the Present Invention

The present invention relates to a needle output buffer mechanism and a syringe comprising the needle output mechanism. In conjunction with the accompanying drawings and preferred embodiments of the present invention, the present invention is further elaborated on the technical means adopted for reaching the intended purpose of the invention.

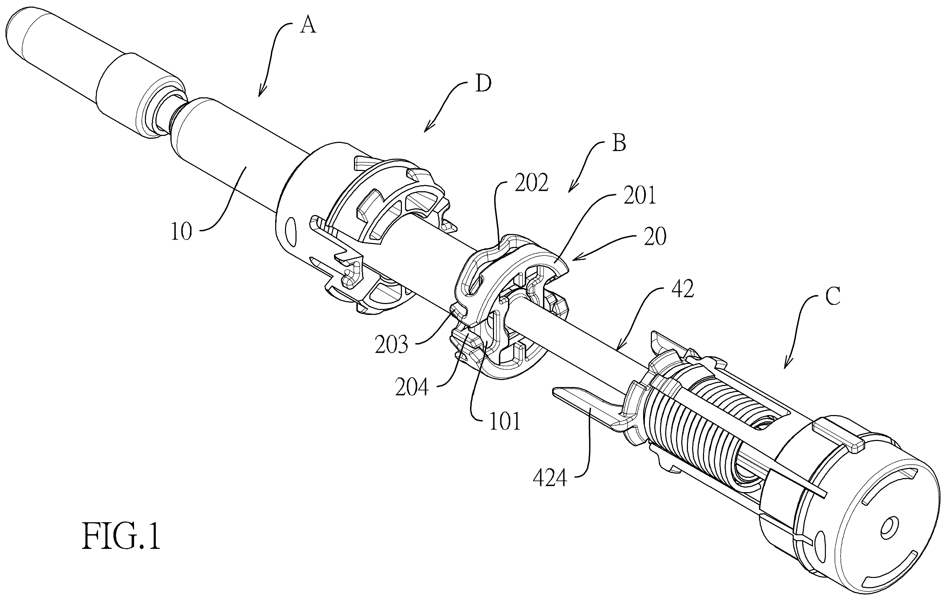

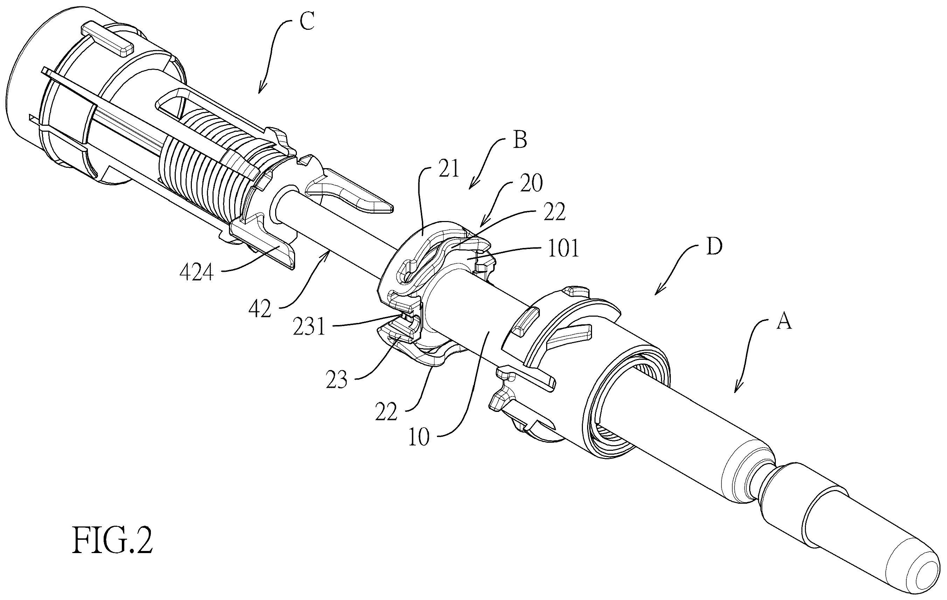

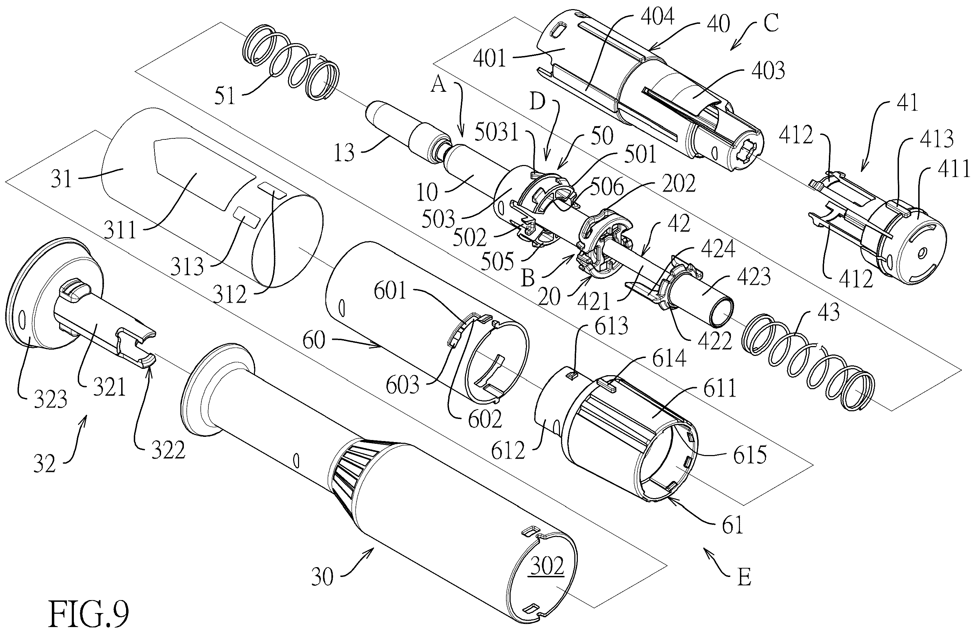

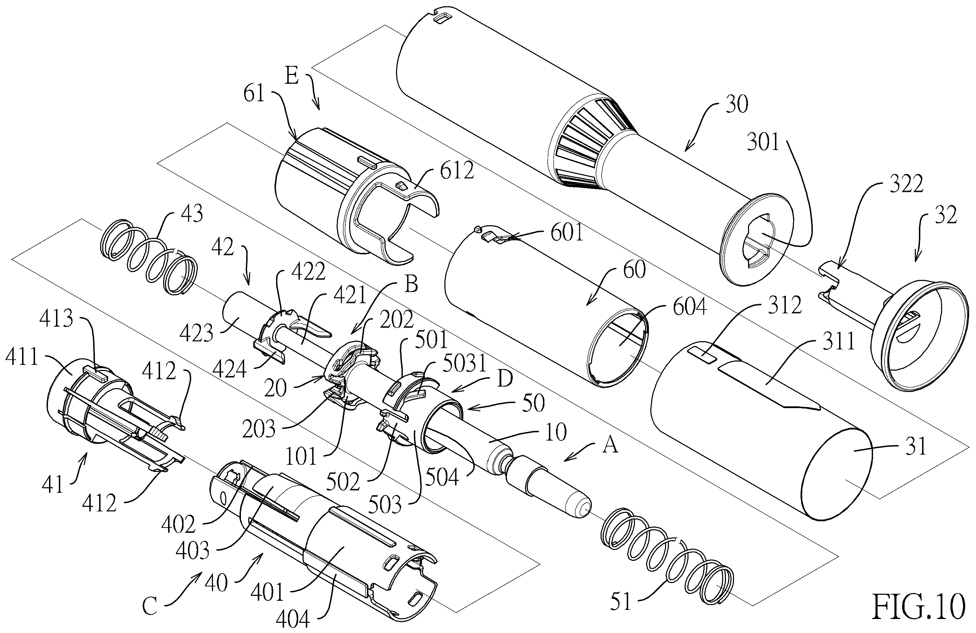

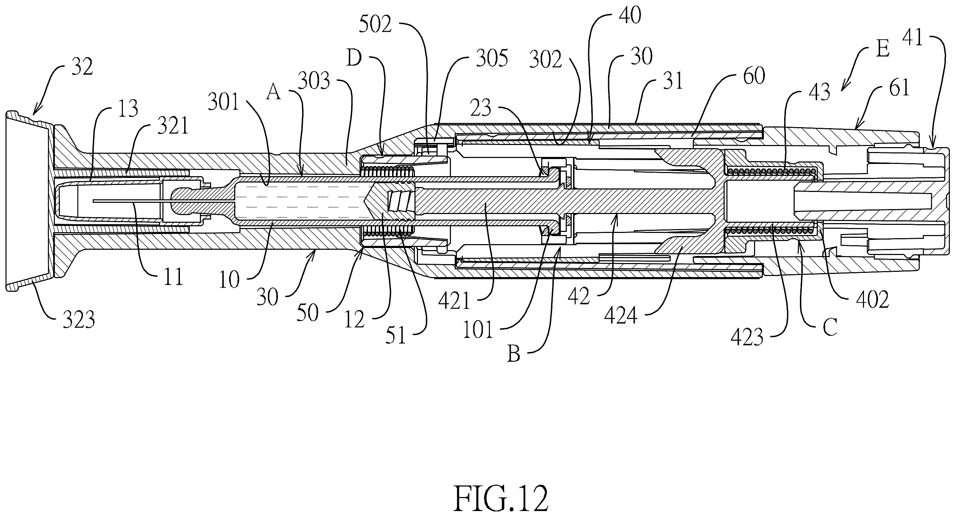

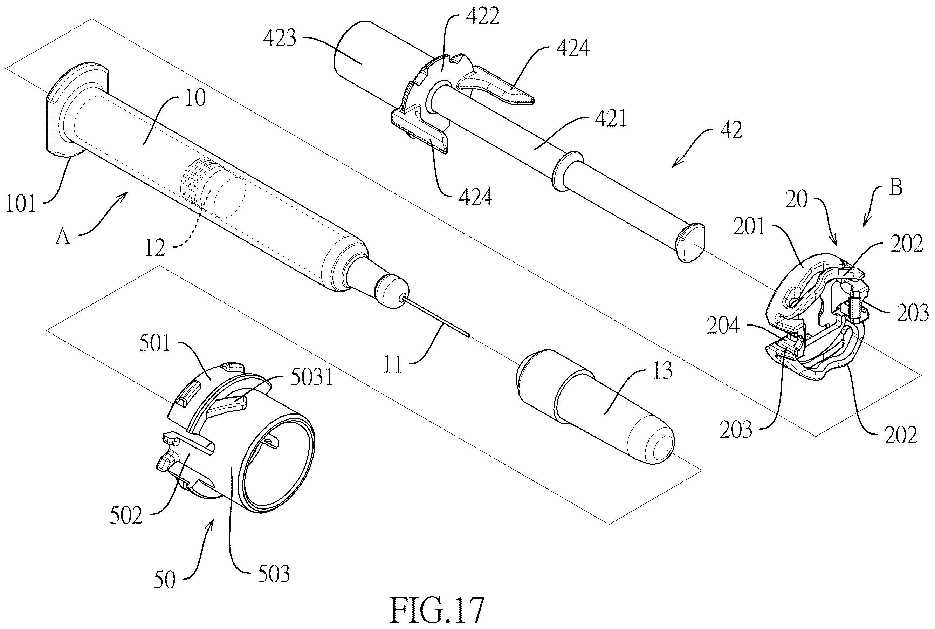

As shown in Figs. 1 to 2 and 7, perspective schematic views of two embodiments of a needle output buffer mechanism B of the present invention being connected with a barrel group A and an injection propulsion mechanism C of a syringe are shown. With reference to Figs. 9, 10, 12 and 16 to 18, said needle output buffer mechanism B is able to be mounted in the syringe having the injection propulsion mechanism C and connected with the barrel group A. The barrel group A includes a barrel 10 with liquid medicine inside. A needle 11 is mounted on a front end of the barrel 10 and a piston is mounted in an interior of the barrel 10. In the embodiments shown in the drawings, two barrel wing portions 101 radially protrude from a rear end of the barrel 10. The injection propulsion mechanism C of the syringe includes an injection push rod 42. The injection push rod 42 is mounted through the needle output buffer mechanism B and is inserted into the barrel 10 from the rear end of the barrel 10. When the injection propulsion mechanism C of the syringe performs a needle ejection action on the barrel group A, the needle output buffer mechanism B is movable along with the barrel group A and provides a buffering effect on the barrel group A in the syringe by using the buffer member 20. Afterwards, the injection push rod 42 of the injection propulsion mechanism C drives the piston 12 in the barrel 10 to output the liquid medicine in the barrel 10 from the needle 11 to perform an injection action.

In addition, said syringe further comprises a needle retracting mechanism D. The needle retracting mechanism D is positioned in front of the needle output buffer mechanism B and is configured to perform a needle retracting action by driving the barrel group A and the needle output buffer mechanism B with the needle retracting mechanism D after the barrel group A has completed the injection action. Meanwhile, the needle retracting mechanism D that is positioned in the front is used as a site for being hit by the needle output buffer mechanism B.

As shown in Figs. 1 to 4 and 5 to 7, said needle output buffer mechanism B comprises a buffer member 20. The buffer member 20 is connected with the barrel group A, and the buffer member 20 includes at least one elastic arm 202. The buffer member 20 is movable along with the barrel group A and generates a buffering elastic force through elastic contact deformation of said elastic arm 202 in the syringe, so that said needle output buffer mechanism B is able to provide buffering elasticity to the barrel group A to eject the needle.

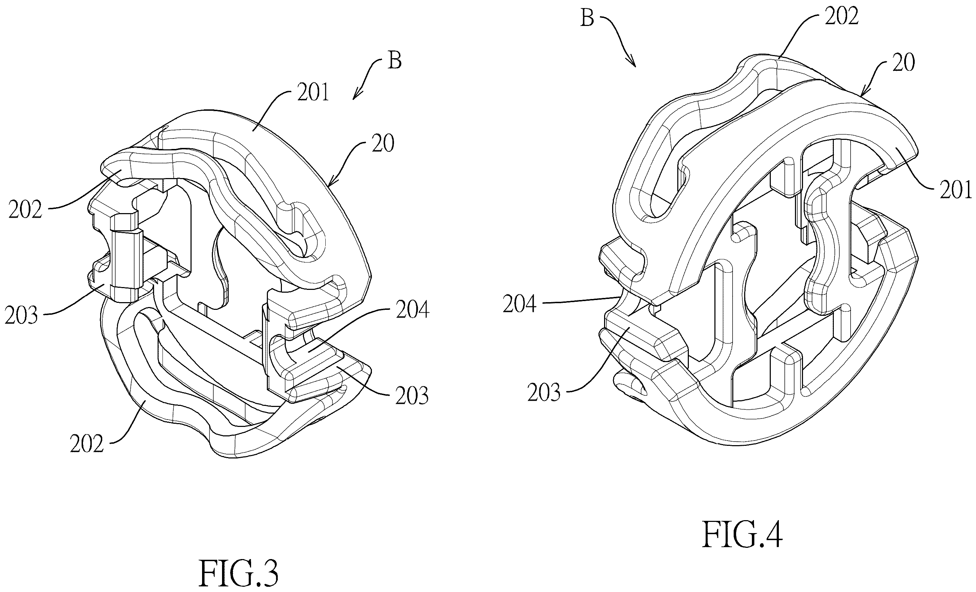

In the embodiment shown in Figs. 3 to 4 and 5 to 6, the buffer member 20 includes a member base 201 and two said elastic arms 202 mounted on a front end of the member base 201. The front end of the member base 201 has two hooking portions 203 arranged symmetrically. The two said elastic arms 202 are symmetrically arranged and disposed by two sides of the two hooking portions 203. Each of said hooking portions 203 may further have an aligning guide slot 204 formed therein. Said aligning guide slots 204 allow a part of the injection propulsion mechanism C to move therethrough, so as to trigger the needle retracting mechanism D positioned in the front to perform the needle retracting action.

In the embodiment shown in Figs. 3 to 4 and 5 to 6, the buffer member 20 is able to abut on the rear end of the barrel 10 with the member base 201 and hook on the two barrel wing portions 101 on the rear end of the barrel 10 with the two hooking portions 203. The two said elastic arms 202 are positioned outside the two barrel wing portions 101 to allow the buffer member 20 to be connected to the rear end of the barrel 10 and the two said elastic arms 202 protrude from front sides of the barrel wing portions 101. In the present embodiment, each of said elastic arms 202 includes a curved concave portion bending backward and two curved convex portions symmetrically disposed on two sides of the curved concave portion and bending forward, and is connected with the member base 201 with another ends, which are opposite to the curved concave portion, of the two curved convex portions. A buffer deformation space exists between said elastic arms 202 and said member base 201, such that the two curved convex portions, which bend and protrude forward, of said elastic arms 202 of the buffer member 20 can be used as a hit portion. A shape and a structure of the elastic arm 202 are not limited to those mentioned above.

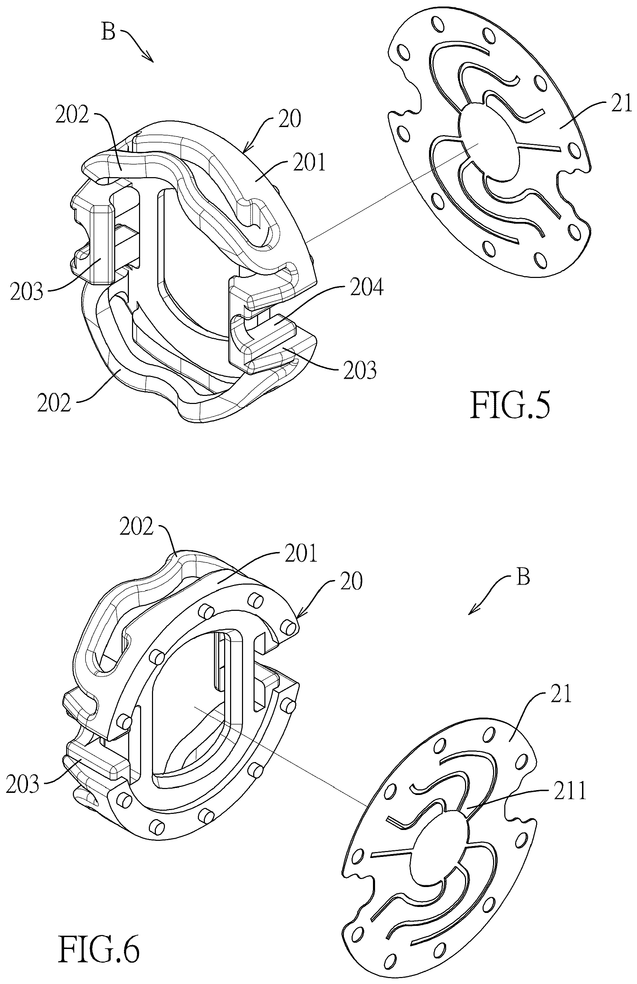

In the embodiment shown in Figs. 5 to 7, the needle output buffer mechanism B includes one said buffer member 20 and a spring plate 21. Basically, a shape and a structure of said buffer member 20 is the same as the buffer member 20 in the previous embodiment. The spring plate 21 is securely mounted on a rear end of the member base 201 of the buffer member 20. The spring plate 21 has a center hole and multiple resilient abutting portions 211 disposed around the center hole. Said resilient abutting portions 211 respectively extend toward the center hole. That is, an end, which faces toward the center hole, of each of said resilient abutting portions 211 is a free end. Each of said resilient abutting portions 211 is able to make sounds when being touched by the injection propulsion mechanism C at different injection positions respectively, thereby using the sounds made by touching the resilient abutting portions 211 at different positions on the spring plate 21 as sound prompt. Otherwise, each of said resilient abutting portions 211 is used to provide resistant force at the different injection positions to the injection propulsion mechanism C, to slow down movement speed of the injection propulsion mechanism C, thereby achieving an effect of slowing down infusion speed.

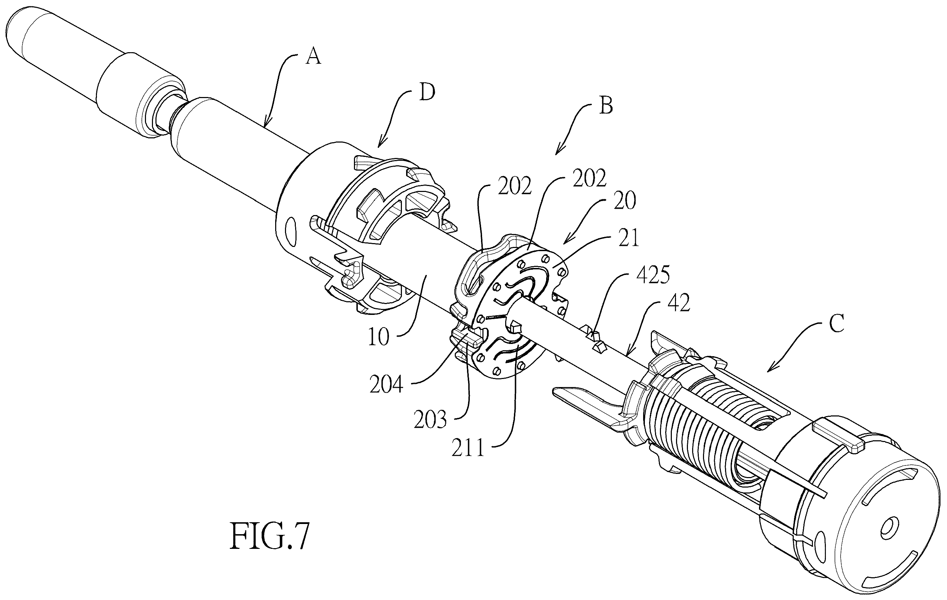

As shown in Fig. 7, the injection propulsion mechanism C includes an injection push rod 42. The injection push rod 42 is mounted through the center hole of the spring plate 21 of the needle output buffer mechanism B and the member base 201 of the buffer member 20, and the injection push rod 42 is able to be driven to push the piston 12 in the barrel 10. Multiple protrusions 425 are formed on an outer surface of the injection push rod 42. The multiple protrusions 425 are disposed at different lengths along an axial direction on the injection push rod 42, and said protrusions 425 are able to touch the resilient abutting portions 211 at corresponding positions respectively to vibrate and make sounds.

Regarding the use of the needle output buffer mechanism B of the present invention in the syringe, as shown in Figs. 1, 2 and 7, it is able to be mounted in the syringe and connected to the rear end of the barrel 10 of the barrel group A inside the syringe. When the barrel group A ejects the needle due to the injection propulsion mechanism C in the syringe, the needle output buffer mechanism B is able to be pushed together with the barrel group A. Moreover, when performing a needle output action, the needle output buffer mechanism B provides the buffering effect on the barrel group A, so as to prevent the barrel group A from being damaged due to collision with internal structure of the syringe. In addition, as shown in Fig. 7, said needle output buffer mechanism B is able to further utilize the spring plate 21 disposed on a rear end of the buffer member 20, so that when the injection push rod 42 pushes the barrel 10 forward to perform the injection, with movement of the injection push rod 42, the protrusions 425 on different positions of the injection push rod 42 is able to touch the resilient abutting portions 211 at corresponding positions of the spring plate 21 respectively to vibrate and make sounds; or/and utilize elastic resistance generated when the resilient abutting portions 211 of the spring plate 21 and the protrusions 425 contact, to slow down moving speed of the injection push rod 42 and achieve the effect of slowing down the infusion speed.

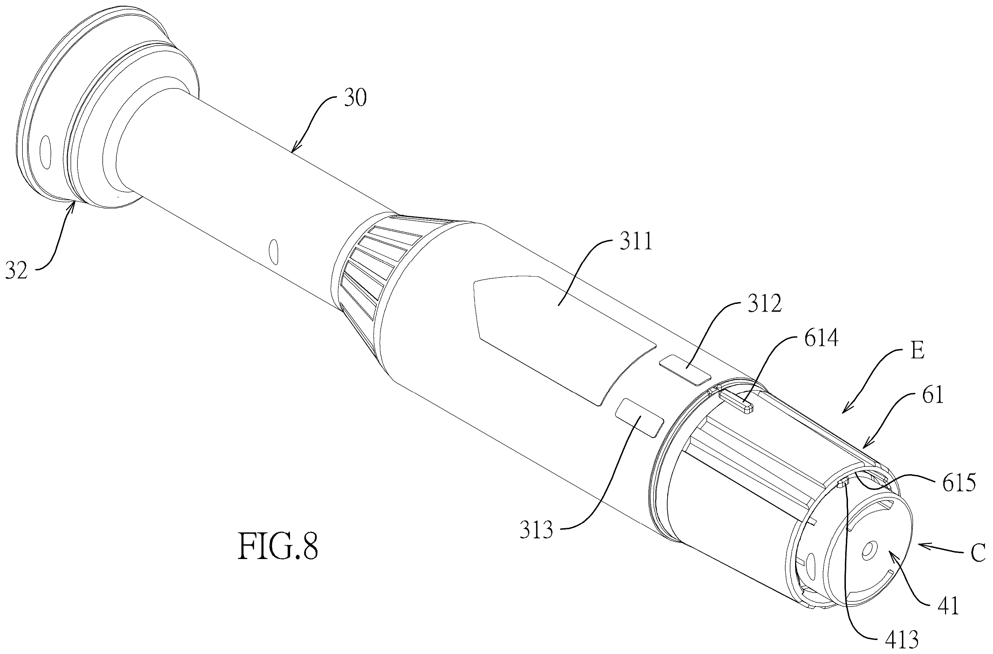

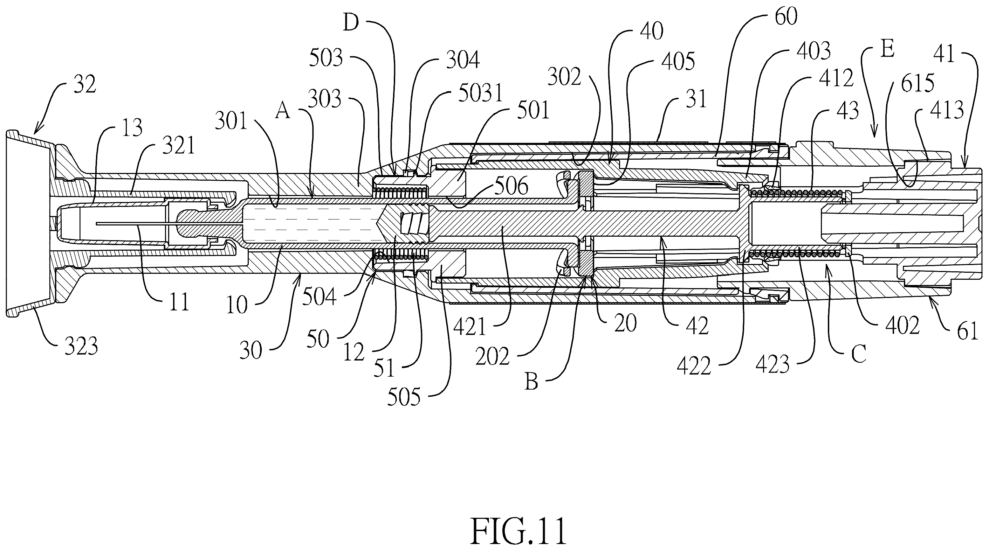

Regarding the syringe of the present invention, a barrel group A is able to be mounted therein for performing the actions of outputting a needle, injection and needle retracting. As shown in Figs. 8 to 12, said barrel group A includes a barrel 10 containing liquid medicine. A needle 11 is mounted on a front end of the barrel 10. A needle sheath 13 may be mounted around the needle 11. A piston 12 is mounted in an interior of the barrel 10 for injecting the liquid medicine from the needle 11. Two barrel wing portions 101 radially protrude from a rear end of the barrel 10. In the present embodiment, the barrel 10 is a component made of a transparent material, but it is not limited to transparent materials.

In the embodiment of the syringe as shown in Figs. 8 to 12, the syringe mainly comprises an outer casing 30, an injection propulsion mechanism C, a needle output buffer mechanism B, and a needle retracting mechanism D. In addition, the syringe may further comprise a safety lock mechanism E.

As shown in Figs. 9 to 12, the outer casing 30 is a hollow component that is axially penetrated from front to rear. An interior of the outer casing 30 has a front accommodating room 301 positioned in the front and a rear accommodating room 302 positioned in the rear. The front accommodating room 301 and the rear accommodating room 302 communicate with each other. An annular retaining wall 303 is formed between the front accommodating room 301 and the rear accommodating room 302. The outer casing 30 has at least one screw groove 304 disposed in a sidewall in the rear accommodating room 302 and disposed adjacent to the annular retaining wall 303 and at least one positioning buckle portion 305 disposed on a rear side of the screw groove 304. The barrel group A is mounted inside the outer casing 30 and is movable forward and backward. In an unused state, the needle 11 of the barrel group A is positioned within the front accommodating room 301 without protruding from a front end of the outer casing 30. The barrel 10 extends from the front accommodating room 301 to the rear accommodating room 302. The rear end, which has the barrel wing portions 101, of the barrel 10 is positioned within the rear accommodating room 302. In the present embodiment, a front section, which has the front accommodating room 301, of the outer casing 30 is transparent, to facilitate a user to observe states of the barrel 10 in the outer casing 30. However, it is not limited thereto, and the front section, which has the front accommodating room 301, of the outer casing 30 may also be an opaque section.

As shown in Figs. 9 to 10, an injection direction indicating mark 311 may be disposed on an outer surface of the outer casing 30, to facilitate the user to identify position of the needle 11 in the syringe. Alternatively, an outer protective layer 31 may also be disposed on the outer surface of the outer casing 30, and said injection direction indicating mark 311 is disposed on the outer protective layer 31.

As shown in Figs. 8 to 12, when the needle sheath 13 is mounted on the needle 11 of the barrel group A, in order to facilitate the user to remove the needle sheath 13, a needle sheath pulling device 32 may be further mounted on the front end of the outer casing 30. The needle sheath pulling device 32 is connected with the needle sheath 13 and is configured to assist the user to remove the needle sheath 13 disposed in the outer casing 30. In the present embodiment, the needle sheath pulling device 32 includes a sleeving portion 321, a hooking portion 322 mounted on a rear end of the sleeving portion 321, and an operation end portion 323 disposed on a front end of the sleeving portion 321. The sleeving portion 321 of the needle sheath pulling device 32 is inserted thereinto from the front end of the outer casing 30 and is mounted around the needle sheath 13. The operation end portion 323 is positioned on an outer side of the front end of the outer casing 30. The hooking portion 322 has two hooks, such that the hooking portion 322 is able to hook on a rear end of the needle sheath 13.

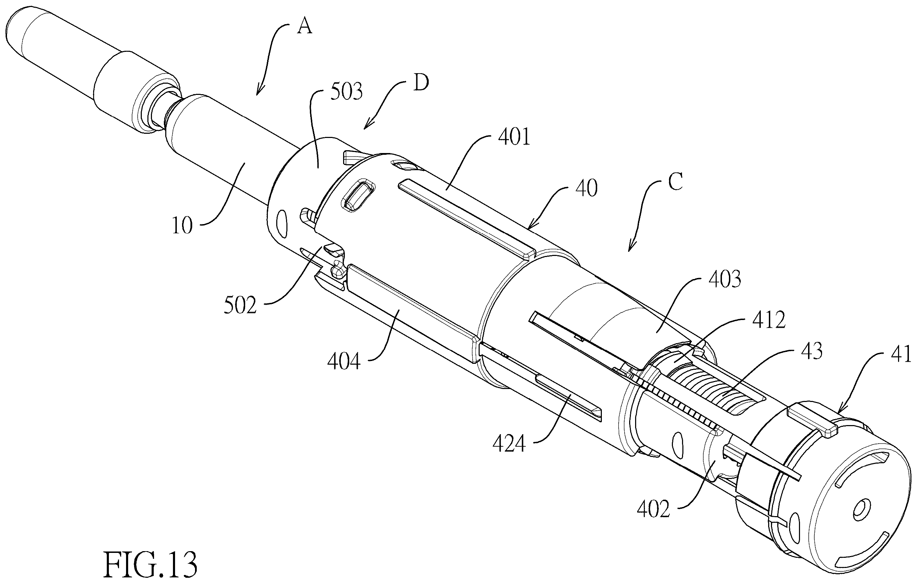

As shown in Figs. 9 to 13, the injection propulsion mechanism C is mounted in the outer casing 30 and protrudes to an outer side of a rear end of the outer casing 30. The injection propulsion mechanism C includes an inner sleeve 40, an injection push rod 42, a rear resilient element 43 and an injection button 41. The injection propulsion mechanism C is operable to eject the barrel group A to perform the needle output action and the injection action.

As shown in Figs. 9 to 13, the inner sleeve 40 is mounted in the rear accommodating room 302 of the outer casing 30. The inner sleeve 40 includes an inner sleeve base 401. At least one stop arm 403 is disposed on a rear end of the inner sleeve base 401, and an axial supporting portion 402 is disposed on the rear end of the inner sleeve base 401. In the present embodiment, the inner sleeve 40 has two said stop arms 403 disposed diametrically opposite each other. The two said stop arms 403 are disposed on two radially opposite sides of the axial supporting portion 402.

As shown in Figs. 9 to 13, the injection push rod 42 is mounted in the inner sleeve 40 and is able to be engaged in place, and the injection push rod 42 is able to be inserted into the barrel 10 to push the piston 12. In the present embodiment, the injection push rod 42 includes a rod part 421, a baffle part 422, a rear rod end 423, and at least one pressing plate 424. The rear rod end 423 is disposed on a rear end of the rod part 421. The baffle part 422 is disposed between the rod part 421 and the rear rod end 423. Said pressing plate 424 protrudes forward from a front side of the baffle part 422 and is disposed beside the rod part 421. In the present embodiment, the injection push rod 42 has two said pressing plates 424. The two said pressing plates 424 are disposed diametrically opposite each other and beside the rod part 421. The rod part 421 of the injection push rod 42 is able to be inserted into the barrel 10 and pushes against the piston 12 with a front end of the rod part 421. The baffle part 422 of the injection push rod 42 is able to be engaged by the stop arms 403 of the inner sleeve 40.

As shown in Figs. 9 to 13, the rear resilient element 43 is mounted in the inner sleeve 40. The rear resilient element 43 is able to accumulate elastic potential energy. When the injection push rod 42 is operated to be released from an engaging state in the inner sleeve 40, the elastic potential energy of the rear resilient element 43 is released to provide ejection power to the injection push rod 42. In the present embodiment, the rear resilient element 43 is a compression spring. The rear resilient element 43 is mounted around an outer side of the rear rod end 423 of the injection push rod 42, and two ends of the rear resilient element 43 abut on the baffle part 422 of the injection push rod 42 and the axial supporting portion 402 of the inner sleeve 40 respectively.

As shown in Figs. 9 to 13, the injection button 41 is connected to a rear end of the inner sleeve 40, is movable forward and backward, and protrudes to the outer side of the rear end of the outer casing 30. The injection button 41 has a pressing end 411 and at least one expanding arm 412 disposed on a front end of the pressing end 411. In the present embodiment, the injection button 41 has two said expanding arms 412 disposed diametrically opposite each other. Each of the expanding arms 412 corresponds to the stop arm 403 of the inner sleeve 40 respectively. The injection button 41 is operable to push against the stop arms 403, which correspond in position thereto, in the inner sleeve 40 to expand through said expanding arms 412, thereby making the baffle part 422 of the injection push rod 42 disengage from the stop arms 403 of the inner sleeve 40 and releasing the injection push rod 42 from the engaging state. In the present embodiment, at least one guiding protrusion 413 may be further disposed on an outer surface of the pressing end 411. Said guiding protrusion 413 is used along with the safety lock mechanism E.

As shown in Figs. 9 to 12 and 14 to 15, in order to ensure the safety of using the injection propulsion mechanism C, the outer casing 30 is mounted to the safety lock mechanism E that is connected with the injection propulsion mechanism C. By limiting movements of the injection propulsion mechanism C with the safety lock mechanism E, when the safety lock mechanism E is in a locked position, the injection propulsion mechanism C is prevented from being accidentally triggered; when the safety lock mechanism E is switched to an unlocked position, the injection propulsion mechanism C is able to be operated to perform the needle output action and the injection action.

In the embodiment shown in Figs. 9 to 12 and 14 to 15, the safety lock mechanism E includes an inner liner tube 60 and a locking ring 61. The inner liner tube 60 is mounted in the rear accommodating room 302 of the outer casing 30. At least one rotating guide groove 601 is defined in a rear section of the inner liner tube 60. Said rotating guide groove 601 has a locking area 602 and an unlocking area 603. The locking ring 61 is a hollow component. A connecting end 612 is defined on a front end of the locking ring 61. At least one rotating guide protrusion 613 is disposed on an outer surface of the connecting end 612. The locking ring 61 has a center hole and at least one guiding groove 615 disposed in a rear section of a hole wall of the center hole. Said guiding groove 615 includes a rotating section and an axially movable section that are connected with each other. The locking ring 61 is disposed behind the outer casing 30. The connecting end 612 protrudes into the rear section of the inner liner tube 60 and is rotatable. Said rotating guide protrusion 613 is disposed in the rotating guide groove 601, which corresponds in position thereto, of the inner liner tube 60. The locking ring 61 is rotatable relative to the inner liner tube 60, to switch a position of said rotating guide protrusion 613 between the locking area 602 and the unlocking area 603 of the rotating guide groove 601. An indicating portion 614 may be further disposed on an outer surface of the locking ring 60. The indicating portion 614 is used to indicate use states of the locking ring 61.

When the rotating guide protrusion 613 is located at the locking area 602, the guiding protrusion 413 of the injection button 41 is limited in the rotating section of the guiding groove 615 of said locking ring 61, such that the locking ring 61 is able to prevent the injection button 41 from moving forward due to being pressed. The injection button 41 is in a locked state, so as to prevent the injection button 41 from being accidentally touched to trigger the injection propulsion mechanism C. When the rotating guide protrusion 613 of the locking ring 61 is rotated to the unlocking area 603, the guiding protrusion 413 of the injection button 41 is switched to the axially movable section of the guiding groove 615 of said locking ring 61 to release the injection button 41 from being limited in the locked state. The axially movable section of said guiding groove 615 provides space for the guiding protrusion 413 of the injection button 41 to move forward, such that the injection button 41 is able to be pressed and move forward.

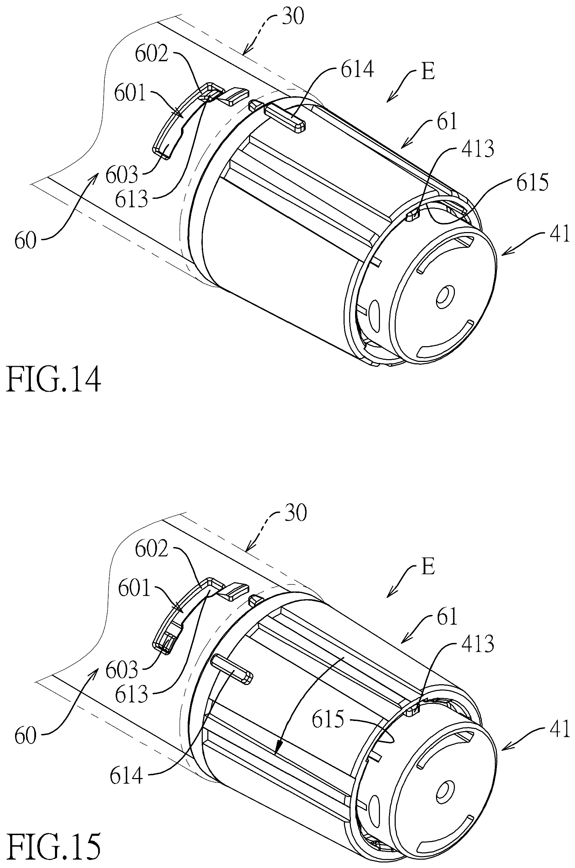

As shown in Figs. 8 to 10, when the syringe includes the safety lock mechanism E, in order to facilitate the users to identify status of the syringe, a lock position mark 312 and an unlock position mark 313 are disposed on an outer surface of the rear end of the outer casing 30; or said lock position mark 312 and said unlock position mark 313 are disposed on the outer protective layer 31 that is on the outer surface of the outer casing 30. As shown in Figs. 14 to 15 and 21 to 22, with the indicating portion 614 that is disposed on the outer surface of the locking ring 61 providing instruction and being used together with said lock position mark 312 and said unlock position mark 313, when the rotating guide protrusion 613 of the locking ring 61 is located in the locking area 602, the indicating portion 614 points at the lock position mark 312; when the rotating guide protrusion 613 of the locking ring 61 is rotated to the unlocking area 603, the indicating portion 614 points at the unlock position mark 313, so as to facilitate the users to recognize the status of the syringe.

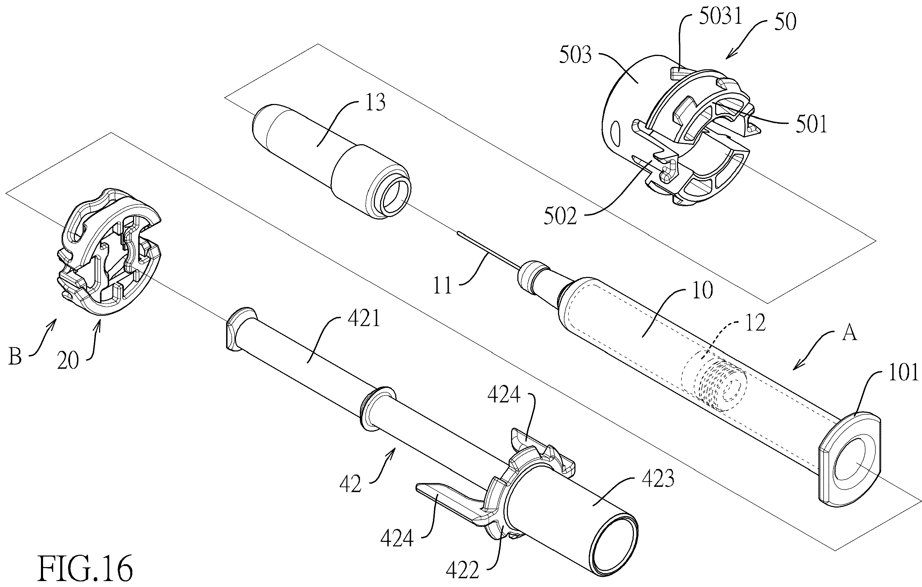

As shown in Figs. 8 to 10, the needle output buffer mechanism B is mounted in the outer casing 30. The needle output buffer mechanism B and the barrel group A are movable together. Specific composed structure of the needle output buffer mechanism B has been disclosed in the description of the needle output buffer mechanism B above, and the part with the same content will not be repeated here. In the present embodiment, as shown in Figs. 16 to 17, the needle output buffer mechanism B is mounted in the inner sleeve 40 of the injection propulsion mechanism C and is connected with the rear end of the barrel 10 of the barrel group A. The rod part 421 of the injection push rod 42 of the injection propulsion mechanism C is mounted through the needle output buffer mechanism B that is mounted on the rear end of the barrel 10 and the rod part 421 is inserted into the barrel 10 to abut on the piston 12. The baffle part 422, which has the pressing plate 424, of the injection push rod 42 is disposed behind the needle output buffer mechanism B. Said pressing plate 424 faces toward the aligning guide slot 204 of the buffer member 20 of the needle output buffer mechanism B.

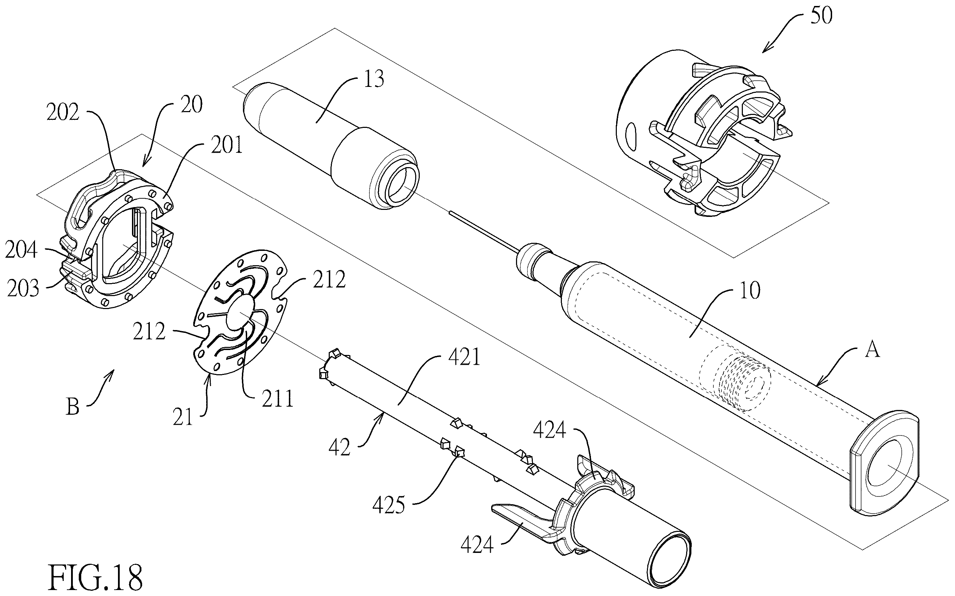

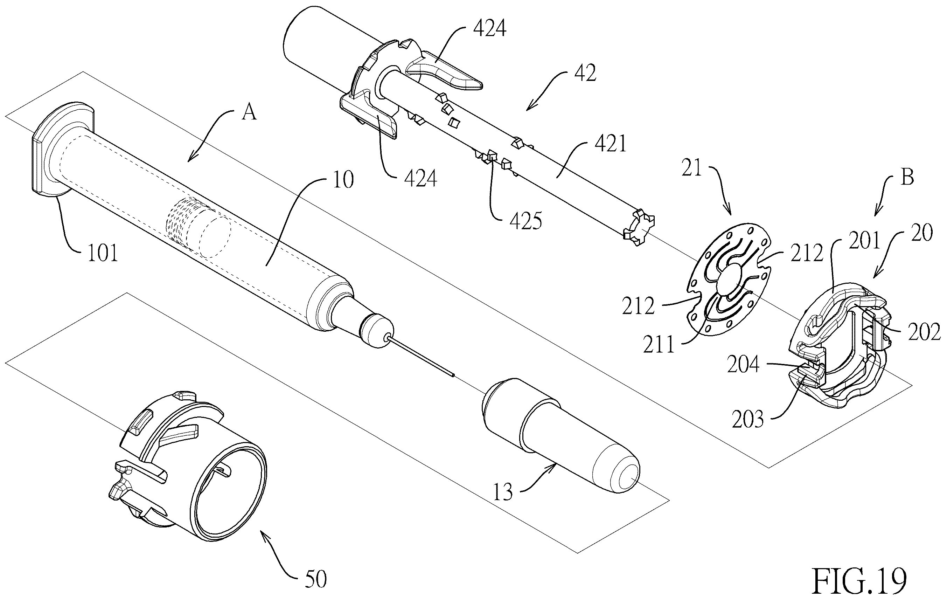

As shown in Figs. 18 to 19, when the spring plate 21 having the multiple resilient abutting portions 211 is mounted on a rear side of the buffer member 20 of the needle output buffer mechanism B, multiple protrusions 425 are additionally disposed on the outer surface of the rod part 421 of the injection push rod 42. The multiple protrusions 425 are disposed at different length positions along an axial direction on the injection push rod 42 or at the same length position along the axial direction, so as to touch the resilient abutting portions 211 at corresponding positions to vibrate and make sounds with each one of said protrusions 425. Moreover, with contact resistance between said protrusions 425 and said resilient abutting portions 211, an effect of slowing down moving speed of the injection push rod 42 is able to be provided.

Furthermore, some medicines may require a slower infusion speed during injection, for instance, to make the patient feel more comfortable, or the medicine itself is designed to require a slower infusion speed. In this circumstance, a number of the protrusions 425 disposed on an outer surface of the rod part 421 of the injection push rod 42 can be increased, thereby increasing a deceleration effect. When the number of the protrusions 425 is increased, contact friction between said protrusions 425 and said resilient abutting portions 211 of the spring plate 21 is also relatively increased. Thus, deceleration effect on the injection push rod 42 would be increased, thereby reducing a speed of the injection push rod 42 pushing the piston 12 in the barrel 10 and reducing the infusion speed. Therefore, a designer can adjust the number and positions of the protrusions 425 according to product design requirement of the syringe. For instance, multiple protrusions 421 are mounted around the rod part 421 at specific length positions along the axial direction on the injection push rod 42 to enhance the deceleration effect. The multiple protrusions 425 are located at the same axial length position. The multiple protrusions 425 touch the multiple resilient abutting portions 211 of the spring plate 21 simultaneously to make sounds together. Rhythm of the prompt sounds would not be mixed.

As described above, with cooperation between the resilient abutting portions 211 of the spring plate 21 and the protrusions 425 of the injection push rod 42, the effects of making prompt sounds and adjusting the infusion speed are able to be provided simultaneously. It is worth mentioning that in other embodiments of the present invention, by changing material and/or shape and structure, the resilient abutting portions 211 of the spring plate 21 and the protrusions 425 of the injection push rod 42 can only cooperate with each other to provide the effect of slowing down the infusion speed and basically do not make any prompt sound that can be heard by the user.

As shown in Figs. 18 to 19, notches 212 may be formed on the spring plate 21 and correspond in position to the aligning guide slots 204 of the buffer member 20. Thus, the pressing plates 424 of the injection push rod 42 are able to enter the aligning guide slots 204 on the buffer member 20 after passing the notches 212 of the spring plate 21, so as to prevent the spring plate 21 on the rear side of the buffer member 20 from interfering with movement of the injection push rod 42.

As shown in Figs. 9 to 12, the needle retracting mechanism D is mounted in the outer casing 30 and is disposed in front of the needle output buffer mechanism B. The needle retracting mechanism D is connected with the barrel group A. The needle retracting mechanism D is operable to drive the barrel group A positioned at a needle output position to move backward until the needle 11 completely returns to a needle retracted position in the outer casing 30, to achieve effects of retracting and protecting a needle tip.

In the embodiment shown in Figs. 9 to 12, the needle retracting mechanism D includes a front fastener 50 and a front resilient element 51. The front fastener 50 is mounted in the rear accommodating room 302 of the outer casing 30 and is able to be engaged in place. A front end of the inner sleeve 40 of the injection propulsion mechanism C is connected with the front fastener 50. The barrel 10 of the barrel group A is mounted through the front fastener 50. The front resilient element 51 is mounted in the front fastener 50 to accumulate elastic potential energy. When the needle retracting mechanism D is operated, an engaging positioning state of the front fastener 50 is able to be released, to allow the front resilient element 51 to release the elastic potential energy and directly or indirectly drive the barrel group A to move backward from the needle output position to the needle retracted position through the front fastener 50.

In the embodiment shown in Figs. 9 to 12, the front fastener 50 includes a fastener base 501 and a screw end 503 disposed on a front end of the fastener base 501. Multiple screw ribs 5031 are disposed on an outer surface of the screw end 503. Multiple ratchet arms 502 that are retractable are disposed on the fastener base 501. Each of said ratchet arms 502 has ratchet teeth. The front fastener 50 is aligned with the screw groove 304, which is disposed adjacent to the annular retaining wall 303, of the outer casing 30 through the screw ribs 5031 of the screw end 503, so as to be mounted in the outer casing 30 in a short-distance radial rotation and connected with the front end of the inner sleeve 40 of the injection propulsion mechanism C through the fastener base 501. Thus, the inner sleeve 40 is movable along with the front fastener 50. The front fastener 50 is engaged in place in the corresponding positioning buckle portion 305 of the outer casing 30 through the ratchet teeth of the ratchet arms 502, and is operable to be released from an engaged state to rotate and move axially in the outer casing 30.

In the embodiment shown in Figs. 9 to 12, the front fastener 50 has an accommodating room 504, an end wall 505 disposed on the rear end of the accommodating room 504, and a fastener bore 506 disposed in the end wall 505 and communicating with the accommodating room 504. The barrel 10 is mounted through the fastener bore 506 and the accommodating room 504. The front resilient element 51 is mounted in the accommodating room 501. Two ends of the front resilient element 51 abut on the annular retaining wall 303 of the outer casing 30 and the end wall 505 respectively and the front resilient element 51 is able to accumulate elastic potential energy. The front fastener 50 is operable to remove restriction of radial rotation, so as to push the front fastener 50 and the inner sleeve 40 connected thereto to rotate by an angle in a reverse direction through the elastic potential energy released by the front resilient element 51 first, so as to allow the screw ribs 5031 to continue axially moving backward after disengaging from the screw groove 304 of the outer casing 30. In the present embodiment, in said needle retracting mechanism D, by passing the aligning guide slots 204 of the buffer member 20 of the needle output buffer mechanism B with the pressing plate 424 of the injection push rod 42 that moves straightly forward, the pressing plate 424 pushes the ratchet arms 502 of the front fastener 50 to retract, so as to make the front fastener 50 to disengage from the positioning buckle portion 305 of the outer casing 30.

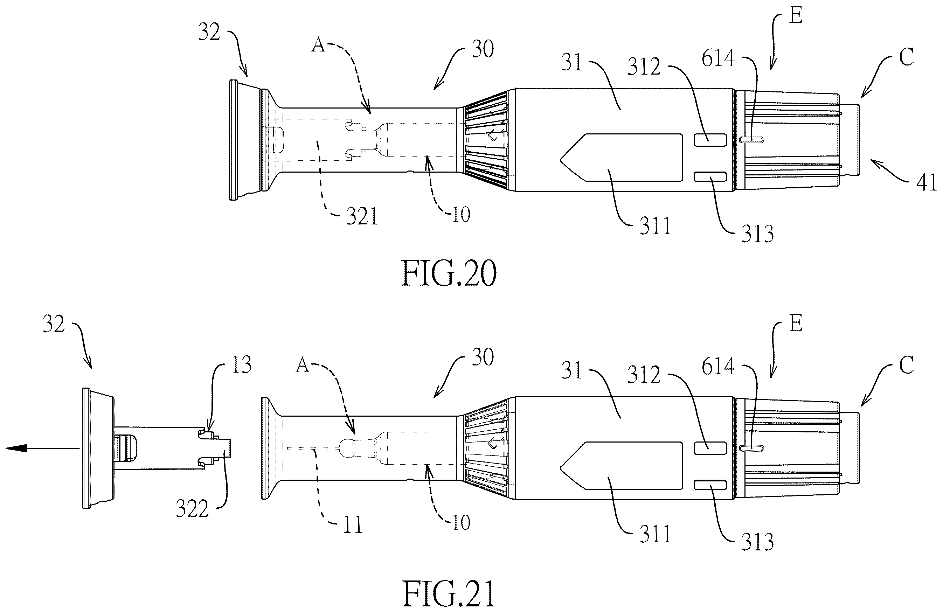

As shown in Figs. 20 to 25, through the overall structural design as described above, the syringe of the present invention is able to assemble the barrel group A in the outer casing 30, and with the needle output buffer mechanism B, the injection propulsion mechanism C and the needle retracting mechanism D that are connected with each other in the outer casing 30, the injection propulsion mechanism C is operable to eject the barrel group A that is connected with the needle output buffer mechanism B to output the needle and to perform the injection action of the liquid medicine. Meanwhile, with the needle output buffer mechanism B providing buffering protection to the barrel 10 of the barrel group A that is ejected and with the needle retracting mechanism D automatically retracting the barrel group A back to the outer casing 30 after completing the injection of the liquid medicine, effects of needle retraction and needle tip protection can be provided. Moreover, in the syringe, the injection propulsion mechanism C and the safety lock mechanism E that are connected with each other may be further mounted in the outer casing 30. By limiting the movements of the injection propulsion mechanism C with the safety lock mechanism E, when the safety lock mechanism E is in the locked position, the injection propulsion mechanism C is prevented from being accidentally triggered; when the safety lock mechanism E is switched to the unlocked position, the injection propulsion mechanism C is able to be operated to perform the needle output action and the injection action.

The embodiment of the syringe shown in Figs. 9 to 12 is used as an example to further illustrate usage scenarios of the syringe of the present invention. With reference to Figs. 20 to 25, an initial state of the syringe is shown. As shown in Figs. 20, 11 and 12, the barrel group A is disposed inside the outer casing 30. The needle sheath pulling device 32 disposed on the front end of the outer casing 30 protrudes into the outer casing 30 and is connected to the needle sheath 13 of the barrel group A. The safety lock mechanism E disposed between the outer casing 30 and the needle propulsion mechanism C protrudes out from the injection button 40 on the rear end of the outer casing 30, so as to prevent the injection propulsion mechanism C from being accidentally triggered.

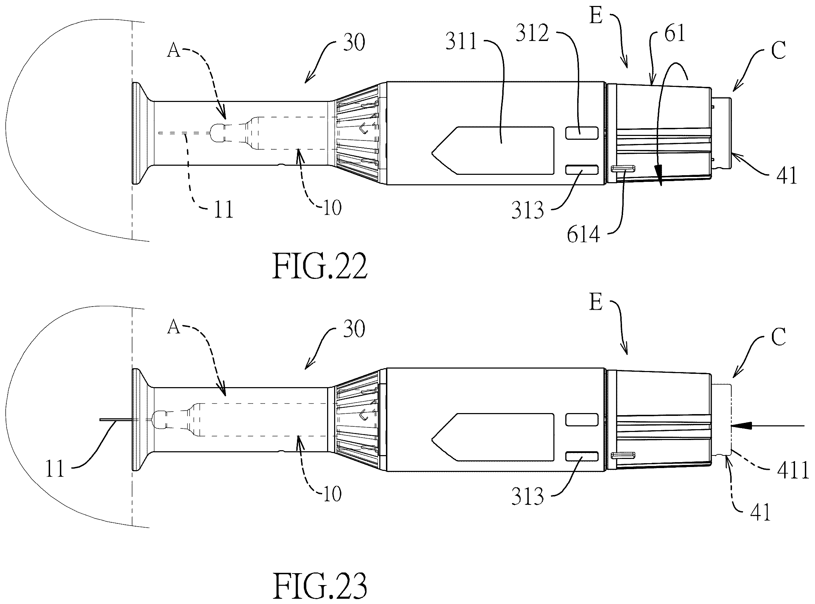

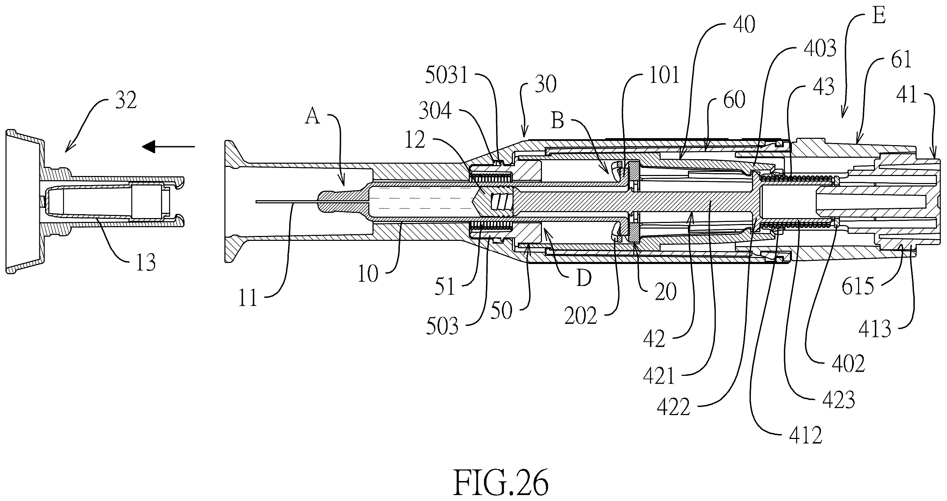

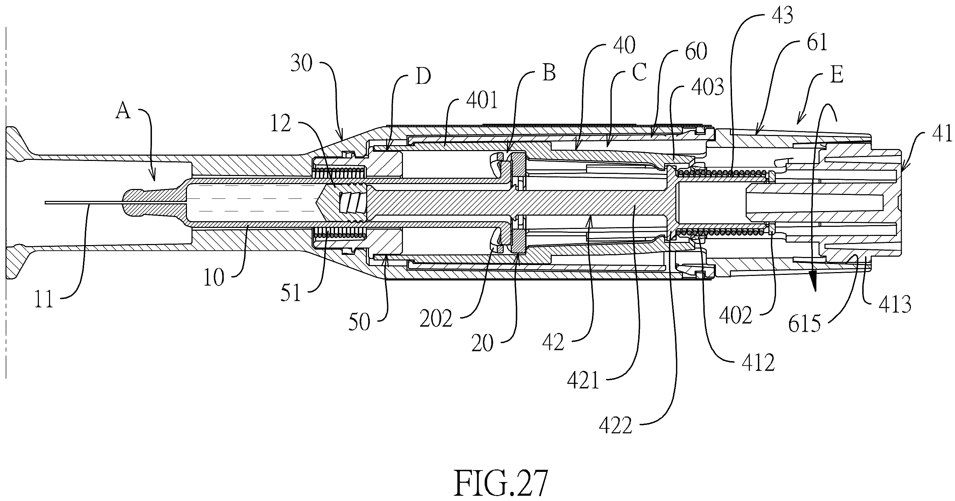

As shown in Figs. 21 and 26, before using the syringe, the needle sheath pulling device 32 is used to remove the needle sheath 13 from the outer casing 30. At this moment, the needle 11 of the barrel group A is still inside the outer casing 30. Afterwards, as shown in Figs. 22 and 27, the front end of the outer casing 30 abuts on a part of a human body to be injected and the locking ring 61 of the safety lock mechanism E is rotated forward, to switch the rotating guide protrusion 613 on the locking ring 61 from the locking area 602 of the rotating guide groove 601 of the inner liner tube 60 to the unlocking area 603. Meanwhile, the guiding protrusion 413 of the injection button 41 is switched to the axially movable section of said guiding groove 615 of the locking ring 61, to release the injection button 41 from the locked state and to provide space for the injection button 41 to move forward.

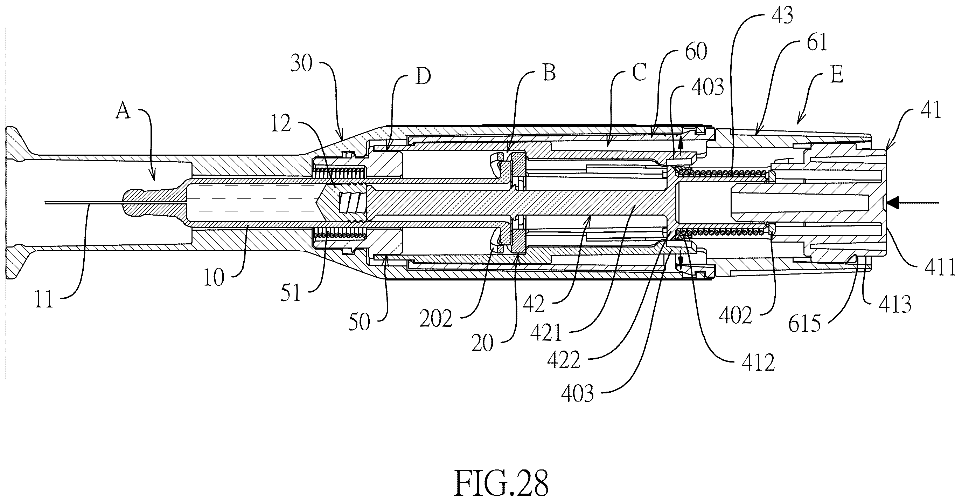

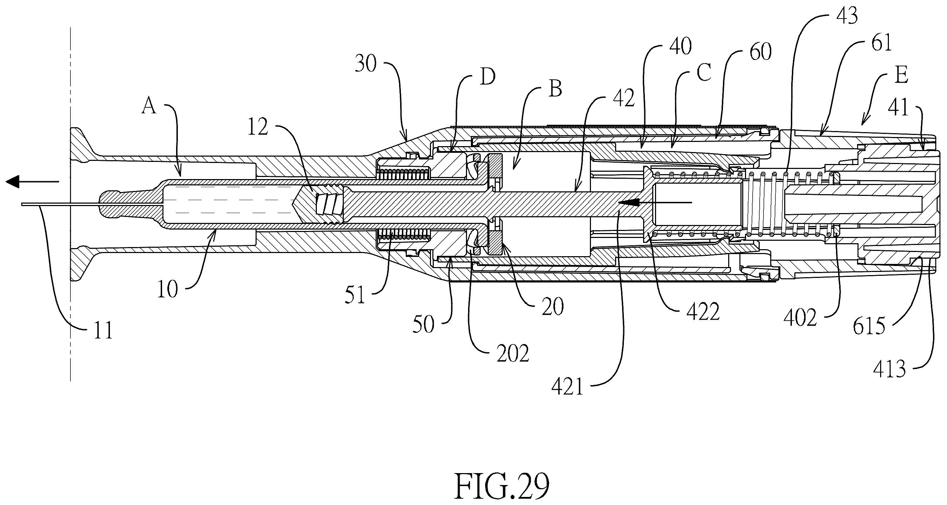

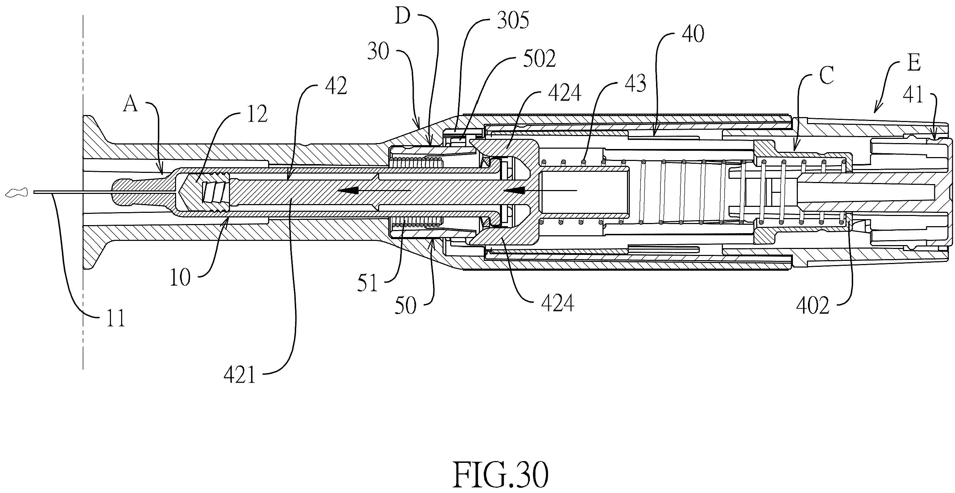

Secondly, as shown in Figs. 23 and 24 and 28 to 30, when the injection button 41 is pressed to move forward, the injection button 41 forwardly pushes the stop arms 403 of the inner sleeve 40 to expand through the expanding arms 412, such that the baffle part 422 of the injection push rod 42 is disengaged from the stop arms 430 of the inner sleeve 40 and is released from the engaging state. Accordingly, the rear resilient element 43 releases the elastic potential energy to eject the injection push rod 42. Moreover, the needle 11 of the barrel group A is pushed out of the front end of the outer casing 30 through the injection push rod 42, such that the needle 11 is able to pierce through the skin of the human body. During a needle ejection process of the barrel group A, with the elastic arms 22 of the needle output buffer mechanism B on the rear end of the barrel 10 resiliently colliding with the needle retracting mechanism D to absorb impact force, it can ensure that the barrel 10 would not be broken due to impact. After the needle 11 has pierced through the skin of the human body, with the elastic potential energy released by the rear resilient element 43 to continue pushing the piston 12 inside the barrel 10 forward through the injection push rod 42, the liquid medicine in the barrel 10 is infused into the human body through the needle 11.

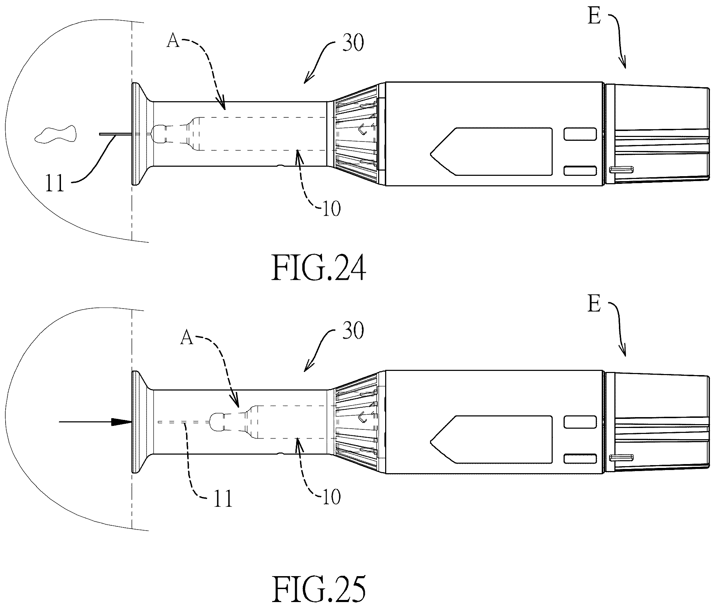

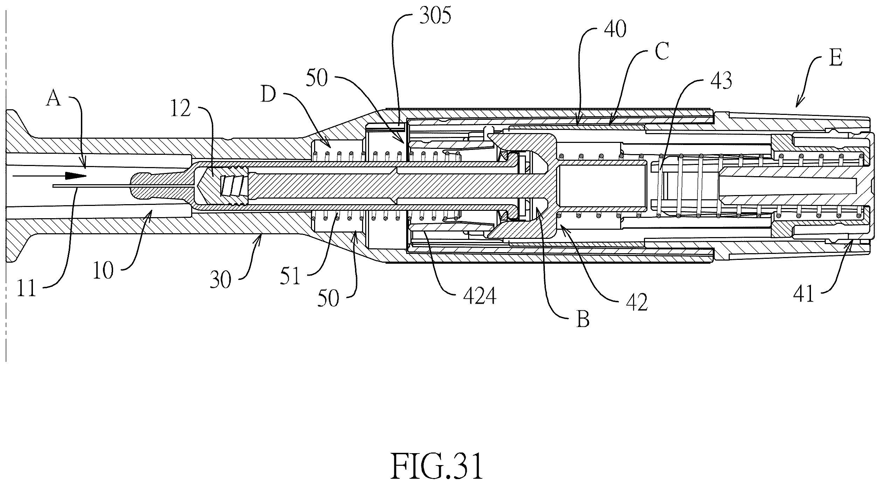

As shown in Figs. 25 and 31, after the infusion is completed, the injection push rod 42 moves forward to the needle output buffer mechanism B due to the elastic potential energy released by the rear resilient element 43. With the pressing plate 424 of the injection push rod 42 passing through the aligning guide slots 204 of the buffer member 20 of the needle output buffer mechanism B and pushing forward on the ratchet arms 502 of the front fastener 50 of the needle retracting mechanism D, the ratchet arms 502 are retracted and disengage from the positioning buckle portion 305 of the outer casing 30. Thus, the elastic potential energy of the front resilient element 51 disposed between the outer casing 30 and the front fastener 50 is released to push the front fastener 50 and the inner sleeve 40 connected thereto to rotate by angle in a reverse direction. After the screw ribs 5031 are disengaged from the screw groove 304 of the outer casing 30 and the elastic potential energy released by the front resilient element 51 pushes the needle retracting mechanism D, the barrel group A connected thereto and the injection propulsion mechanism C to axially move backward, the needle 11 of the barrel group A moves back into the outer casing 30 and the needle retracting action is completed.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A needle output buffer mechanism (B), mounted in a syringe; the needle output buffer mechanism (B) connected with a barrel group (A) and **characterized in that**: when the barrel group (A) is ejected to output a needle (11) by an injection propulsion mechanism (C) in the syringe, the needle output buffer mechanism (B) and the barrel group (A) are movable together; the needle output buffer mechanism (B) comprising:
a buffer member (20) connected with the barrel group (A) and the buffer member (20) including at least one elastic arm (202); the buffer member (20) providing buffering elasticity to the barrel group (A) that is ejected to output the needle (11) through elastic contact of the elastic arm (202) in the syringe.

2. The needle output buffer mechanism (B) as claimed in claim 1, **characterized in that**: the buffer member (20) includes a member base (201) and two elastic arms (202) symmetrically mounted on a front end of the member base (201); the front end of the member base (201) has two hooking portions (203) arranged symmetrically, the two elastic arms (202) are symmetrically arranged and disposed by two sides of the two hooking portions (203); the two hooking portions (203) hook on two barrel wing portions (101) on a rear end of a barrel (10) of the barrel group (A); the two elastic arms (202) are positioned outside the two barrel wing portions (101) and protrude from front sides of the barrel wing portions (101).

3. The needle output buffer mechanism (B) as claimed in claim 2, **characterized in that**: each of the elastic arms (202) includes a curved concave portion bending backward and two curved convex portions symmetrically disposed on two sides of the curved concave portion and bending forward; the elastic arm (202) is connected with the member base (201) through the two curved convex portions.

4. The needle output buffer mechanism (B) as claimed in claim 3, **characterized in that**: each of the hooking portions (203) has an aligning guide slot (204) formed therein; a part of the injection propulsion mechanism (C) passes through the aligning guide slots (204).

5. The needle output buffer mechanism (B) as claimed in any one of claims 2 to 4, **characterized in that**: the needle output buffer mechanism (B) further includes a spring plate (21); the spring plate (21) is securely mounted on a rear end of the member base (201); the spring plate (21) has a center hole and multiple resilient abutting portions (211) disposed around the center hole and extending toward the center hole; each of the resilient abutting portions (211) is touchable by a positionally respective one of protrusions (425) on an outer surface of an injection push rod (42) of the injection propulsion mechanism (C).

6. A syringe, a barrel group (A) containing liquid medicine mounted therein, **characterized in that** the syringe comprises:
an outer casing (30); an interior thereof having a front accommodating room (301) and a rear accommodating room (302) arranged front to back and communicating with each other; the barrel group (A) mounted in the outer casing (30) and being movable in the front accommodating room (301) and the rear accommodating room (302);
an injection propulsion mechanism (C) mounted in the rear accommodating room (302) of the outer casing (30) and protruding out from a rear end of the outer casing (30); the injection propulsion mechanism (C) disposed behind the barrel group (A) and being operable to eject the barrel group (A) forward from the front accommodating room (301) of the outer casing (30) to output a needle (11) and inject the liquid medicine;
a needle output buffer mechanism (B) mounted in the rear accommodating room (302) of the outer casing (30); the needle output buffer mechanism (B) connected to a rear end of a barrel (10) of the barrel group (A) and being movable along with the barrel group (A); the needle output buffer mechanism (B) including a buffer member (20); the buffer member (20) including at least one elastic arm (202); the buffer member (20) providing buffering elasticity to the barrel group (A) that is ejected to output the needle (11) through elastic contact of the elastic arm (202) in the syringe;
a needle retracting mechanism (D) mounted in the rear accommodating room (302) of the outer casing (30) and positioned in front of the needle output buffer mechanism (B); the barrel group (A) passing through the needle retracting mechanism (D); after the injection propulsion mechanism (C) is operated to eject the barrel group (A) and the needle output buffer mechanism (B) connected thereto to complete a needle ejection action and an injection action of the liquid medicine, the needle retracting mechanism (D) is triggered to drive the barrel group (A) and the needle output buffer mechanism (B) that is connected with the barrel group (A) and the injection propulsion mechanism (C) move backward until the needle (11) of the barrel group (A) returns to the outer casing (30).

7. The syringe as claimed in claim 6, **characterized in that**: the buffer member (20) includes a member base (201) and two elastic arms (202) symmetrically mounted on a front end of the member base (201); the elastic arm (202) includes a curved concave portion bending backward and two curved convex portions symmetrically disposed on two sides of the curved concave portion and bending forward; the elastic arms (202) are connected with the member base (201) through the two curved convex portions; the front end of the member base (201) has two hooking portions (203) arranged symmetrically, the two elastic arms (202) are symmetrically arranged and disposed by two sides of the two hooking portions (203); the two hooking portions (203) hook on two barrel wing portions (101) on a rear end of the barrel (10); the two elastic arms (202) are positioned outside the two barrel wing portions (101) and protrude from front sides of the barrel wing portions (101).

8. The syringe as claimed in claim 6 or 7, **characterized in that**: the syringe further comprises a safety lock mechanism (E); the safety lock mechanism (E) is mounted in the outer casing (30) and is connected with the injection propulsion mechanism (C); the safety lock mechanism (E) is switchable between a locked state and an unlocked state of the injection propulsion mechanism (C).

9. The syringe as claimed in claim 6 or 7, **characterized in that**: the injection propulsion mechanism (C) includes an inner sleeve (40), an injection push rod (42), a rear resilient element (43) and an injection button (41);
the inner sleeve (40) is mounted in the rear accommodating room (302) of the outer casing (30); the inner sleeve (40) includes an inner sleeve base (401); at least one stop arm (403) is disposed on a rear end of the inner sleeve base (401), and an axial supporting portion (402) is disposed on the rear end of the inner sleeve base (401);
the injection push rod (42) is mounted in the inner sleeve (40) and is able to be inserted into the barrel (10) to push a piston (12); the injection push rod (42) includes a rod part (421), a rear rod end (423) disposed behind the rod part (421), a baffle part (422) disposed between the rod part (421) and the rear rod end (423), and at least one pressing plate (424) disposed in front of the baffle part (422) and beside the rod part (421); the baffle part (422) is able to be engaged by the stop arm (403); the pressing plate (424) is able to trigger the needle retracting mechanism (D) to perform a needle retracting action through the needle output buffer mechanism (B);
the rear resilient element (43) is mounted in the inner sleeve (40) and is disposed between the baffle part (422) and the axial supporting portion (402); when the injection push rod (42) is engaged in the inner sleeve (40), the rear resilient element (43) accumulates elastic potential energy;
the injection button (41) is connected to the rear end of the inner sleeve (40), is movable forward and backward, and protrudes to an outer side of the rear end of the outer casing (30); the injection button (41) has a pressing end (411) and at least one expanding arm (412) disposed on a front end of the pressing end (411); the injection button (41) is operable to push the stop arm (403) to expand through said expanding arm (412) to release the injection push rod (42) from an engaging state; the rear resilient element (43) releases the elastic potential energy to eject the injection push rod (42).

10. The syringe as claimed in claim 9, **characterized in that**: the syringe further comprises a safety lock mechanism (E); the safety lock mechanism (E) is mounted in the outer casing (30) and is connected with the injection propulsion mechanism (C); the safety lock mechanism (E) is switchable between a locked state and an unlocked state of the injection propulsion mechanism (C);
at least one guiding protrusion (413) is disposed on the pressing end (411) of the injection button (41) of the injection propulsion mechanism (C);
the safety lock mechanism (E) includes an inner liner tube (60) and a locking ring (61); the inner liner tube (60) is mounted in the outer casing (30); at least one rotating guide groove (601) is defined in a rear section of the inner liner tube (60); the rotating guide groove (601) has a locking area (602) and an unlocking area (603); a connecting end (612) is defined on a front end of the locking ring (61); at least one rotating guide protrusion (613) is disposed on an outer surface of the connecting end (612); the locking ring (61) has at least one guiding groove (615); the guiding groove (615) includes a rotating section and an axially movable section that are connected with each other; the locking ring (61) is disposed behind the outer casing (30); the connecting end (612) of the locking ring (61) protrudes into the rear section of the inner liner tube (60) and is rotatable; the rotating guide protrusion (613) is disposed in the rotating guide groove (601), which corresponds in position thereto, of the inner liner tube (60); when a location of the rotating guide protrusion (613) is switched between the locking area (602) and the unlocking area (603), the injection propulsion mechanism (C) is switched between the locked state and the unlocked state through cooperation between the rotating guide groove (601) of the locking ring (61) and the rotating guide protrusion (613) of the injection button (41).

11. The syringe as claimed in claim 9, **characterized in that**: the needle retracting mechanism (D) includes a front fastener (50) and a front resilient element (51); the front fastener (50) is mounted in the rear accommodating room (302) of the outer casing (30) and is able to be engaged in place; a front end of the inner sleeve (40) of the injection propulsion mechanism (C) is connected with the front fastener (50); the barrel (10) of the barrel group (A) is mounted through the front fastener (50); the front resilient element (51) is mounted in the front fastener (50) to accumulate elastic potential energy; when the needle retracting mechanism (D) is triggered by the pressing plate (424) of the injection push rod (42), an engaging positioning state of the front fastener (50) is able to be released to allow the front resilient element (51) to release the elastic potential energy and drive the front fastener (50), the barrel group (A), the needle output buffer mechanism (B) and the injection propulsion mechanism (C) to move backward to a needle retracted position.

12. The syringe as claimed in claim 11, **characterized in that**: an annular retaining wall (303) is formed between the front accommodating room (301) and the rear accommodating room (302) of the outer casing (30); the outer casing (30) has at least one screw groove (304) disposed in a sidewall in the rear accommodating room (302) and disposed adjacent to the annular retaining wall (303) and at least one positioning buckle portion (305) disposed on a rear side of the screw groove (304);
the front fastener (50) of the needle retracting mechanism (D) includes a fastener base (501) and a screw end (503) disposed on a front end of the fastener base (501); multiple screw ribs (5031) are disposed on an outer surface of the screw end (503); multiple ratchet arms (502) that are retractable are disposed on the fastener base (501); each of the ratchet arms (502) has ratchet teeth; the front fastener (50) is mounted in the outer casing (30) through cooperation of the screw ribs (5031) and the screw groove (304); the fastener base (501) of the front fastener (50) is connected with the front end of the inner sleeve (40) of the injection propulsion mechanism (C); the inner sleeve (40) is movable along with the front fastener (50); the front fastener (50) is engaged in place in the corresponding positioning buckle portion (305) of the outer casing (30) through the ratchet teeth of the ratchet arms (502); the ratchet arms (502) are able to be triggered by the pressing plate (424) of the injection push rod (42) to remove restriction of radial rotation of the front fastener (50), and the front fastener (50) and the inner sleeve (40) are pushed to rotate with the elastic potential energy released from the front resilient element (51), so as to allow the screw ribs (5031) to disengage from the screw groove (304) of the outer casing (30).

13. The syringe as claimed in claim 7, **characterized in that**: the needle output buffer mechanism (B) further includes a spring plate (21); the spring plate (21) is securely mounted on a rear end of the member base (201); the spring plate (21) has a center hole and multiple resilient abutting portions (211) disposed around the center hole and extending toward the center hole; each of the resilient abutting portions (211) is touchable by a positionally respective one of protrusions (425) on an outer surface of an injection push rod (42) of the injection propulsion mechanism (C).
